# EUROPEAN PATENT APPLICATION

(11) **EP 4 574 106 A1**
(43) Date of publication of application: **25.06.2025**
(21) Application number: 23218136.2
(22) Date of filing: 19.12.2023
(51) Int. Cl.: A61F 7/00

(54) **TEMPERATURE REGULATION APPARATUS AND ASSOCIATED METHODS**

(71) Applicant: The Surgical Company International B.V., 3821 AH Amersfoort (NL)
(72) Inventor: TEUNISSEN, Berend Jan, 3821 AH Amersfoort (NL); SYRI, Tommy Andreas Christian, 3821 AH Amersfoort (NL); GARCÍA MUÑOZ, José Ignacio García, 3821 AH Amersfoort (NL)
(74) Representative: Hindles Limited

(57) **Abstract**

In summary, there is provided a temperature regulation apparatus (1, 101) comprising a forced gas temperature regulation blanket (2, 102) for providing temperature-regulated gas to a subject, the forced gas temperature regulation blanket comprising: a first blanket portion (4, 104) comprising: an inflatable region (10, 110), the inflatable region comprising a gas permeable region; and an inlet port (8, 108) for receiving temperature-regulated gas into the inflatable region, a second blanket portion (6, 106) comprising at least two at least partially mutually separable planar sections (14A, 14B, 114A, 114B), each configured for covering a limb of a subject.

## Description

### Field of the invention

The present invention relates to temperature regulation of subjects during medical procedures, particularly procedures carried out when the subject is in the lithotomy position, and similar.

### Background to the invention

It is known to provide subjects with blankets or covers during medical procedures to limit any decrease in the subject's temperature during the procedure. It is further known to actively regulate the temperature of subjects during procedures, for example using temperature regulated gas (e.g. air).

Some procedures require the subject's limbs (e.g. legs) to be spread apart. For example, procedures which are carried out when the subject is in the lithotomy position, which allows examinations and procedures involving the pelvis and lower abdomen. In the lithotomy position a subject will lie down on their back with their hips at or close to the edge of an examination table or bed, and with their legs raised and spread, and supported by with support structures such as stirrups, typically at the knee and/or ankle.

It is in this context that the present inventions have been devised.

### Summary of the invention

In accordance with an aspect of the present invention, there is provided a temperature regulation apparatus comprising a forced gas temperature regulation (e.g. warming) blanket for providing (e.g. configured to provide) temperature-regulated gas to a subject, the forced gas temperature regulation (e.g. warming) blanket comprising:
a first blanket portion comprising:
   an inflatable region, the inflatable region comprising a gas permeable region; and
   an inlet port for receiving temperature-regulated gas into the inflatable region,
a second blanket portion comprising at least two at least partially mutually separable planar sections, each configured for (e.g. at least partially) covering (e.g. wrapping around) a limb of a subject.

Temperature regulation apparatuses comprising portions which in turn comprise at least two partially mutually separable planar sections are particularly helpful, as these sections can be used to cover (or wrap around) a subject's limbs (particularly a subject's legs), even when the subject's limbs are not adjacent to each other. In procedures which require the subject to be in the lithotomy position (or other positions requiring the legs to be spread apart) a blanket not having mutually separable planar sections will not typically cover the subject's legs. This can lead to a decrease in the subject's temperature during the procedure, which can have adverse effects. Accordingly, the provision of mutually separable planar sections provides a way to cover the subject's limbs without the requirement for further blankets or other equipment, and thus limits the risk of the subject's temperature decreasing during procedures where the limbs are not adjacent to each other.

The gas may be air. For example, the gas may comprise a mixture of gases including nitrogen and oxygen, e.g. in the proportions typically found in ambient air. The gas may comprise oxygen. The gas may comprise inert gases. The forced gas temperature regulation blanket will typically be a forced gas warming blanket (e.g. for the supply of warm gas (e.g. air)) however this is not required, and in some instances the forced gas temperature regulation blanket may be a forced gas cooling blanket (e.g. for the supply of cool gas (e.g. air)). For example, the forced gas temperature regulation blanket may be a warming blanket in most medical procedures, as subjects' temperatures tend to drop during procedures and the provision of a forced gas warming blanket can mitigate this. However, in some cases, such as fever, the forced gas temperature regulation blanket may be a cooling blanket configured to help to reduce the subject's temperature when it is too high. It will be understood that "warm", "warming", "warm air" etc may all refer to temperatures and air/gas temperatures which are great enough to increase or maintain the subject's temperature (e.g. where the subject is a typical human, temperatures above 31 °C, e.g. above 35 °C, e.g. above 38 °C). Similarly, it will be understood that "cool", "cooling", "cool air" etc may all refer to temperatures and air/gas temperature which are low enough to reduce the subject's temperature.

The forced gas temperature regulation (e.g. warming) blanket may be a forced air temperature regulation (e.g. warming) blanket.

The apparatus may be an apparatus for providing (e.g. configured to provide) temperature-regulated gas to a subject when the subject is in the lithotomy position. For example, the first blanket portion may be sized and shaped to correspond with the size and shape of a typical adult human upper body (e.g. from the top of the head to generally the hip region), or for example may have greater dimensions and the second blanket portion (e.g. the planar sections) may be sized and shaped to correspond with the size and shape of a typical adult human lower body (e.g. leg, e.g. from generally the hip region to the foot), or may for example have greater dimensions. The planar sections may be sized and shaped to correspond with the size and shape of the typical adult human leg, for example, they may be sized and shaped such that they (e.g. each) may be used to cover a respective leg, optionally such that they (e.g. each) may be used to wrap around a respective leg.

In some examples, it is helpful to cover the subject's upper leg (e.g. thigh), or to wrap the blanket around the upper leg (e.g. the thigh) only and not to cover the full leg. This is because the upper leg (e.g. thigh) makes up the greatest proportion of the leg, and contains the largest muscles and blood vessels. It has been found that providing temperature regulated gas to the upper leg (e.g. thigh) and torso is sufficient to regulate the temperature of the subject and that the additional benefit of providing temperature regulated gas to the full leg is not always required. As such, material costs can be reduced by providing a second blanket portion (e.g. planar sections) that is sized and shaped to correspond with the typical adult human upper leg (e.g. thigh). Furthermore, it is easier to move a planar section which is sized and shaped to cover an upper leg (rather than a full leg) out of the way, should it not be needed for a particular procedure.

The second blanket portion (e.g. the planar sections) may be sized and shaped to correspond with the size and shape of a typical adult human upper leg (e.g. thigh) (or may for example have greater dimensions). The planar sections may be sized and shaped to correspond with the size and shape of the typical adult human upper leg (e.g. thigh), for example, they may be sized and shaped such that they (e.g. each) may be used to cover a respective upper leg (e.g. thigh), optionally such that they (e.g. each) may be used to wrap around a respective upper leg (e.g. thigh).

For example, the temperature regulation apparatus (including the first and second blanket portions) may have a length of more than 2 meters, e.g. more than 2.2 meters, e.g. more than 2.5 meters, typically less than 3.5 meters, e.g. less than 2.8 meters (e.g. as measured when the inflatable region is not inflated). The temperature regulation apparatus may have a width of more than 0.75 meters, e.g. more than 1 meter, e.g. more than 1.25 meters, typically less than 2 meters, e.g. less than 1.5 meters (e.g. as measured when the inflatable region is not inflated).

Alternatively, where the second blanket portion is sized and shaped to correspond to the size and shape of a typical adult human leg (e.g. thigh), the temperature regulation apparatus (including the first and second blanket portions) may have a length of more than 1.5 meters, e.g. more than 1.8 meters, e.g. more than 2 meters, typically less than 3 meters, e.g. less than 2.5 meters (e.g. as measured when the inflatable region is not inflated).

The first blanket portion may have a length of more than 1 meter, e.g. more than 1.1 meter, e.g. more than 1.3 meters, typically less than 1.8 meters, e.g. less than 1.5 meters (e.g. as measured when the inflatable region is not inflated). The first blanket portion may have a width of more than 0.75 meters, e.g. more than 1 meter, e.g. more than 1.25 meters, typically less than 2 meters, e.g. less than 1.5 meters (e.g. as measured when the inflatable region is not inflated). The second blanket portion may have a length of more than 1 meter, e.g. more than 1.1 meter, e.g. more than 1.3 meters, typically less than 1.8 meters, e.g. less than 1.5 meters. The second blanket portion may have a width of more than 0.75 meters, e.g. more than 1 meter, e.g. more than 1.25 meters, typically less than 2 meters, e.g. less than 1.5 meters.

Alternatively, where the second blanket portion is sized and shaped to correspond to the size and shape of a typical adult human leg (e.g. thigh), the second blanket portion may have a length of more than 0.5 meters, e.g. more than 0.7 meters, e.g. more than 0.8 meters, typically less than 1 meter, e.g. less than 0.9 meters.

The temperature regulated gas (e.g. air) may be warmed (e.g. warm) gas (e.g. air). The temperature regulated gas (e.g. air) may be cooled (e.g. cool) gas (e.g. air). The temperature regulated gas (e.g. air) may be gas (e.g. air) having a temperature between 30 °C and 45 °C, e.g. between 36 °C and 40 °C, e.g. approximately 38 °C. In some examples, the temperature regulated gas (e.g. air) may be gas (e.g. air) having a temperature between 41 °C and 45 °C, e.g. between 42 °C and 44 °C, for example approximately 43 °C. In some examples, the temperature regulated gas (e.g. air) may be gas (e.g. air) having a temperature between 30 °C and 34 °C, e.g. between 31 °C and 33 °C, for example approximately 32 °C The temperature regulated gas (e.g. air) may be gas (e.g. air) having a temperature selected such that when the gas (e.g. air) is directed towards the subject in use, the temperature of the gas on reaching the subject is between 35 °C and 39 °C, e.g. between 36 °C and 38 °C, e.g. approximately 37 °C.

The second blanket portion may be configured such that separation of the mutually separable planar sections defines (and/or expands) a space in the second blanket portion, the space overlapping a longitudinal axis of the second blanket portion (e.g. a longitudinal axis of the second blanket portion may be parallel to, optionally coincident with, a longitudinal axis of the space).

It will be understood that, in use, the apparatus would typically extend in a direction parallel to the horizontal plane. It will be further understood that the first and second blanket portions thus have a thickness perpendicular to the horizontal plane. Furthermore, it will be understood that the apparatus (and the first and second blanket portions) extend in first and second horizontal directions, wherein the first horizontal direction is perpendicular to the second horizontal direction. The apparatus may be elongate, in that it is longer than it is wide (e.g. and typically both longer and wider than it is thick). Accordingly, where the longitudinal axis is referred to, this is typically the axis parallel to the greatest dimension of the apparatus (e.g. the length).

A second blanket portion configured such that the separation of the mutually separable planar sections defines (and/or expands) a space in the second blanket portion, where that space overlaps a longitudinal axis of the second blanket portion, provides an advantage where procedures are carried out on a subject in the lithotomy position (or other positions in which the subject's legs are spread apart). In particular, this allows a space for a medical professional to access the subject between the subject's legs, and particularly to access the pelvic region, without the apparatus getting in the way. This is particularly helpful in procedures and examinations involving reproductive organs, urology, and gastrointestinal systems.

The first blanket portion typically extends from a first end, along the length of the apparatus, towards an intermediate location. The second blanket portion typically extends from the intermediate location, along the length of the apparatus away from the first blanket portion and towards a second end. The first and second blanket portions may be directly connected to each other at the intermediate location. Alternatively, the apparatus may comprise an intermediate blanket portion between the first and second blanket portions, at the intermediate location.

The second blanket portion may comprise connected but mutually separable planar sections. The second blanket may comprise mutually separable planar sections with a space defined therebetween. Where the second blanket portion comprise connected but mutually separable planar sections, the apparatus can still be used for procedures where the subject's limbs are not spread apart, without the separable planar sections getting in the way, or getting folded up and stuck under the subject.

The apparatus (e.g. at least the first blanket portion) may comprise an underbody. It will be understood that an underbody is a blanket configured to be positioned at least partially (optionally wholly) beneath a subject in use. The first blanket portion may be configured to be positioned at least partially (optionally wholly) beneath a subject in use.

Advantageously, where the apparatus (e.g. the first blanket portion) is provided as an underbody, it does not get in the way of the medical professional, and can still supply temperature regulated gas (e.g. air) to the subject from below. This is particularly helpful where the subject is in the lithotomy position, where an apparatus positioned over a subject may restrict access to the subject by the medical professional.

The planar sections may (e.g. each) be configured for covering (e.g. wrapping around) the leg of a subject. The second blanket portion may be configured such that there is a space provided between the at least two at least partially mutually separable planar sections. Alternatively, the second blanket portion may comprise a separation structure configured to allow easy mutual separation of the at least two at least partially mutually separable planar sections. For example, the second blanket portion (optionally the separation structure) may comprise at least one first line of weakness. The at least one line of weakness may be configured to form (e.g. define a boundary between) the at least two at least partially mutually separable planar sections. For example, the at least one line of weakness may be configured such that tearing along the at least one line of weakness will separate the mutually separable planar sections.

The at least one first line of weakness may be provided generally along (e.g. parallel to) the longitudinal axis of the apparatus. The at least one first line of weakness may be provided between two of the at least two mutually separable planar sections. The at least one first line of weakness may extend from the intermediate location towards the second end. The second blanket portion may comprise one or more additional line of weakness (for example generally formed as with the at least one first line of weakness). The one or more additional lines of weakness may be provided in an upper region of the second blanket portion (e.g. relatively closer to the first blanket portion than to the lower end of the blanket). One or more (e.g. two) of the one or more additional lines of weakness may each extend from a longitudinal outermost edge of the second blanket portion towards the longitudinal axis of the blanket. One or more of the one or more additional lines of weakness may extend across the longitudinal axis and towards a longitudinal outermost edge of the second blanket portion. The one or more additional lines of weakness may be configured such that tearing along the additional lines of weakness provides a greater range of movement in the planar sections, such that the planar sections can be more readily used to at least partially cover (optionally to be at least partially wrapped around) the limb of a subject.

The provision of a second blanket portion comprising a line of weakness as described above provides the advantage that a user of the apparatus does not need to use an additional tool (such as a pair of scissors) to separate the mutually separable planar sections.

The at least one first line of weakness may comprise (e.g. be) a line section where the apparatus (e.g. the second blanket portion) has reduced thickness, e.g. when compared to other parts of the apparatus (e.g. the second blanket portion). The at least one first line of weakness may comprise (e.g. be) a line of perforations. The at least one first line of weakness may comprise a pull-tab which, when pulled, cuts through the apparatus (e.g. the second blanket portion) across the line of weakness. The at least one first line of weakness typically will typically be a section (e.g. line) which is configured to allow a user to separate the planar sections more easily than they would separate any other two parts of the first blanket portion (not connected via a line of weakness). It will be understood that the at least one first line of weakness need not be a straight line and may instead be curved, or optionally may follow a u-shaped path, for example. The at least one first line of weakness may comprise two lines of weakness, optionally three or more lines of weakness. The at least one first line of weakness may comprise one or more line sections.

The second blanket portion may comprise a fold line configured to allow the planar sections to be folded towards the torso of the subject. The fold line may comprise a line where the apparatus (e.g. the second blanket portion, optionally the apparatus at the intermediate location) has a reduced thickness (e.g. when compared to other parts of the apparatus (e.g. the second blanket portion). The fold line may comprise a seam. The fold line may comprise a crease (e.g. a pre-folded section). The second blanket portion may comprise a pleat, the pleat comprising the fold line.

The provision of a fold line makes it easier to move the planar sections away from the subject's upper legs in a controlled and predictable way, which can be helpful when the medical professional needs access to the limb (e.g. the leg, optionally the upper leg). By providing a fold line, a known point of folding is used, which is preferable to a blanket portion without a fold line. This is because a user of the apparatus where the blanket portion has no fold line will tend to fold the blanket in a less controlled way, which can mean that the blanket tends to fold back to its original position and/or can get caught on other equipment.

The fold line may comprise at least one second line of weakness. The at least one second line of weakness may be configured to allow at least one (e.g. each) of the planar sections to be detached (e.g. removed) from the blanket. The at least one second line of weakness may comprise (e.g. be) a line where the apparatus (e.g. the second blanket portion) has reduced thickness, e.g. when compared to other parts of the apparatus (e.g. the second blanket portion). The at least one second line of weakness may comprise (e.g. be) a line of perforations. The at least one second line of weakness may comprise a pull-tab which, when pulled, cuts through the apparatus (e.g. the second blanket portion) across the line of weakness. The at least one second line of weakness typically will typically be a section (e.g. line) which is configured to allow a user detach (e.g. remove) the planar sections from the apparatus more easily than they would detach (e.g. remove) any other part of the first blanket portion. It will be understood that the at least one second line of weakness need not be a straight line and may instead be curved, for example. The at least one second line of weakness may comprise one or more line sections.

Where the fold line comprises a second line of weakness, this can be used to conveniently detach (e.g. remove) the planar sections from the blanket, for example where they are not needed (and will not be needed for the specific procedure) or are in the way.

The temperature regulation apparatus may comprise a first layer and a second layer. The first layer may be a gas (e.g. air) permeable layer (e.g. the first layer may be more gas permeable than the second layer). The second layer may be a gas (e.g. air) impermeable layer. The forced gas temperature regulation (e.g. warming) blanket may be configured to allow (e.g. force) gas (e.g. air) to leave the inflatable region (e.g. via the first layer) and, for example, to thereby be directed towards a subject. The apparatus may be configured to provide temperature regulated gas (e.g. air) to the subject because it includes a gas (e.g. air) permeable first layer which allows gas (e.g. air) to leave the inflatable region and thereby be directed towards the subject. It will be understood that a blanket having an inflatable region and a gas (e.g. air) permeable layer via which air is forced to leave, as described herein, is a forced gas (e.g. air) temperature regulation (e.g. warming) blanket.

This provides a convenient way of forming a temperature regulation apparatus wherein temperature regulated gas (e.g. air) can be provided between the first and second layers in use, and can then leave via the gas permeable layer.

The first layer and the second layer may be connected to each other via at least one seal extending from the first layer to the second layer (e.g. at least partially around the perimeter of each layer, optionally around the full perimeter of each layer, optionally around the perimeter of the first blanket portion) such that the first layer the second layer, and the seal, thereby form (e.g. define) the inflatable region (e.g. an internal chamber for receiving temperature regulated gas). The temperature regulation apparatus may comprise one or more further seals within the inflatable region, connecting the first layer and the second layer, to thereby limit the distance by which the first and second layers may be separated when the inflatable region is inflated. The first and second layers may be separated by a maximum distance of at least 3 centimetres, e.g. at least 5 centimetres, e.g. at least 10 centimetres, typically less than 30 centimetres, e.g. less than 25 centimetres, e.g. less than 20 centimetres (e.g. at the inflatable region, when inflated).

A seal may comprise a direct connection between the first and second layers (such that the layers are directly connected to each other at the location of the seal). Alternatively, the seal may comprise an indirect connection via an intermediate element.

The gas permeable region may extend across the first blanket portion and the second blanket portion. The gas permeable region may extend across the full area of the inflatable region, e.g. across the full first layer. The first layer is typically a gas permeable layer. In use, the gas permeable layer is typically arranged to face the subject. A gas permeable region (or layer) provides a convenient way to allow temperature regulated gas to flow out of the inflatable region and to be directed towards the subject with a controlled rate of flow, and in a way that is distributed across a defined area.

The second layer may comprise a reflective outer coating. A reflective outer coating is helpful in insulating the subject and will reflect heat back towards the subject in use. This is particularly helpful in this instance where the apparatus comprises mutually separable planar sections which may be used to cover (or which may be wrapped around) the limbs of a subject, and where the apparatus is an underbody. This is because the reflective outer coating will be generally beneath the subject and facing away from the subject, and when the planar sections are used to cover (or are wrapped around) the subject's limbs, the reflecting outer coating will again be facing away from the subject's limbs. In other words, where the planar sections are wrapped around a limb to form a sleeve-like structure, the reflective outer coating is most conveniently positioned on the outside of the sleeve facing away from the limb (rather than the inside of the sleeve facing towards the limb).

The apparatus (e.g. the second blanket portion) may comprise one or more auxiliary planar section(s). The one or more auxiliary planar section(s) may be (e.g. at least partially, optionally wholly) mutually separable from each other planar section. The one or more auxiliary planar section may be formed of an additional layer, typically an impermeable (e.g. gas or air impermeable) layer, optionally a layer comprising a reflective outer coating. It will be understood that a gas (or air) impermeable layer will still permit some gas to pass therethrough at sufficiently high pressures, and such a layer should be less permeable to gas (e.g. air) than a gas (e.g. air) permeable layer.

In some cases, it is helpful to completely detach the mutually detachable planar sections from the apparatus, for example where a medical professional requires access to an upper leg during a procedure and the planar section is in the way. In such cases, it can be helpful to provide auxiliary planar sections, which may be brought into later use, when access to the upper leg is no longer required.

The first blanket portion may comprise one or more seals formed between the first layer and the second layer, the one or more seals at least partially bounding the inflatable region. The one or more seals may be configured such that the inflatable portion is comprised (e.g. wholly) within the first blanket portion, e.g. and such that the second blanket portion is uninflatable (e.g. does not comprise an inflatable portion).

Providing an apparatus with an inflatable region in a first blanket portion and an uninflatable second blanket portion (having mutually separable planar sections) is particularly helpful, because if the second blanket portion (and thus the planar sections) were inflatable, they would take up additional space. This would make it more difficult to cover the subject's limbs with the planar sections, or to wrap the sections around the limbs. In cases where the legs are spread apart to allow a medical professional to access the subject's pelvic region (such as where the subject is in the lithotomy position) it is particularly helpful to have planar sections which do not inflate and therefore do not obstruct the medical professional's access.

Furthermore, the inventors have surprisingly found that providing planar sections which do not inflate does not prevent proper temperature regulation of the subject. In particular, where the apparatus is an underbody, temperature regulated gas (e.g. air) may leave the underbody via the permeable layer (e.g. region) and be directed towards the subject, where it then flows away from the subject. The gas flowing away from the subject in the region of the planar sections is caught and directed by the planar sections towards the subject's limbs. Temperature regulation of the subject's upper body and limbs is thus achieved.

The second blanket portion may comprise an adhesive layer between the first layer and the second layer. This helps to prevent the two layers from separating and moving independently under the flow of gas (even where they are not otherwise fixed in place).

The first blanket portion may comprise one or more connectors, e.g. one or more tie strips. The second blanket portion may comprise one or more connectors, e.g. one or more tie strips. Each of the one or more connectors may be configured to secure a respective planar section at least partially over (e.g. around, optionally to) the limb of a subject. The one or more connectors may comprise one or more tie strips. The one or more connectors may comprise one or more press-studs. The one of connectors may comprise one or more buttons with corresponding button holes. The one or more connectors may comprise one or more Velcro (TM) sections, etc.

It is helpful to provide an apparatus as described hereinabove, wherein the planar sections comprise connectors, as these allow for the planar sections to be used to cover a limb of a subject (optionally wrapped around a limb of the subject) and fixed in place, either by connecting one part of the planar section to another part of the planar section, or by connecting a part of the planar section to something else (e.g. furniture, optionally the stirrups of a lithotomy chair). This helps to prevent the planar sections from moving around during use.

The forced gas temperature regulation (e.g. warming) blanket may comprise a detachable (e.g. removable) section boarded by at least one third line of weakness.

The at least one third line of weakness may be configured such that the detachable (e.g. removable) section can be detached (e.g. removed) from the blanket to thereby form an aperture in the blanket for positioning between the limbs of a subject. Alternatively or additionally, the forced gas temperature regulation (e.g. warming) blanket may have an aperture defined therein, the aperture for positioning between the limbs of a subject. The at least one third line of weakness may bound a region intermediate the planar sections.

The at least one third line of weakness may comprise (e.g. be) a line where the apparatus (e.g. the second blanket portion) has reduced thickness, e.g. when compared to other parts of the apparatus (e.g. the second blanket portion). The at least one third line of weakness may comprise (e.g. be) a line of perforations. The at least one third line of weakness may comprise a pull-tab which, when pulled, cuts through the apparatus (e.g. the second blanket portion) across the line of weakness. The at least one third line of weakness typically will typically be a section (e.g. line) which is configured to allow a user detach (e.g. remove) the detachable (e.g. removable) section from the apparatus more easily than they would detach (e.g. remove) any other part of the first blanket portion. It will be understood that the at least one third line of weakness need not be a straight line and may instead be curved, or optionally may follow a u-shaped path, for example. The at least one third of weakness may comprise two lines of weakness, optionally three or more lines of weakness. The at least one third line of weakness may comprise one or more line sections.

The provision of such a third line of weakness allows a user to further separate the planar sections and/or to detach a section between the planar sections to thereby provide more space for a medical professional to access a subject in use.

The apparatus may be configured such that, when separated, a region of a planar section closer (e.g. a longitudinal edge of a planar section closest) to the space (e.g. the space overlapping a longitudinal axis of the second blanket portion) can be moved over a limb, e.g. and towards a region of a planar section further (e.g. a longitudinal edge of a planar section furthest) from the space (e.g. the space overlapping a longitudinal axis of the second blanket portion), for example to thereby form a sleeve which wraps around the limb. In an example, the apparatus may be configured such that where the region of the planar section closer to the space is initially be beneath the subject, it may be passed between the legs, e.g. and the planar section may moved to cover the top of a leg, and the region (that was originally) closer to the space may be moved over the limb, for example to meet the region of the planar section further from the space (e.g. to form a sleeve which wraps around the leg).

The first blanket portion may comprise a locating marker configured to indicate where the blanket should be positioned relative to a subject.

This provides a quick and efficient way for a user of the blanket to determine that the blanket is positioned correctly with respect to the subject's body; that it is the right size for the subject; and that the gas-permeable layer is provided such that temperature-regulated gas will be directed towards the subject in use.

According to a further aspect of the invention, there is provided a method of supplying temperature-regulated gas to a subject using a temperature regulation apparatus as described herein. The method may comprise providing the forced gas temperature regulation (e.g. warming) blanket beneath a subject. The method may comprise positioning the forced gas temperature regulation (e.g. warming) blanket beneath a subject, e.g. with reference to a locating marker. The method may comprise causing temperature-regulated gas (e.g. air) to be supplied into the inflatable region (e.g. via the inlet port), e.g. to thereby cause the inflatable region to inflate. The method may comprise supplying temperature-regulated gas (e.g. air) into the inflatable region (e.g. via the inlet port), e.g. to thereby cause the inflatable region to inflate.

The inlet port is typically configured to be connectable to a supply of temperature-regulated gas. For example, the inlet port may comprise a connector for connecting with a supply of temperature-regulated gas (e.g. via a conduit).

The method may comprise causing temperature-regulated gas (e.g. air) to be directed towards a subject, optionally via the gas-permeable layer (e.g. by forcing air to leave via the gas-permeable layer). The method may comprise directing temperature-regulated gas (e.g. air) towards a subject, optionally via the gas-permeable layer.

The method may comprise separating the mutually separable planar sections, optionally by tearing along a line of weakness. The method may comprise (e.g. at least partially) covering the limb of a subject with a (e.g. each) planar section. The method may comprise wrapping a (e.g. each) planar section (e.g. at least partially) around the limb of a subject.

The method may comprise folding the apparatus (e.g. the second blanket portion), optionally along a fold line. For example, the method may comprise folding one or more (e.g. each) planar section away from the limbs of a subject, along a fold line. The method may comprise detaching one or more (e.g. each) planar section from the apparatus (e.g. the second blanket portion), optionally by tearing along a line of weakness.

It may be that the second blanket portion comprises one or more connectors (e.g. tie strips). It may be that the one or more connectors are (e.g. each) configured to secure a planar section in place, for example (e.g. at least partially) over or around the limb of a subject. The method may comprise securing a planar section (e.g. at least partially) around a limb of the subject. The method may comprise securing a planar section (e.g. at least partially) around both a limb and at least one further structure (e.g. a piece of furniture such as a hospital bed of lithotomy chair). The method may comprise covering the (e.g. each) leg of a subject with a (e.g. respective) planar section. The method may comprise wrapping a (e.g. each) planar section around a (e.g. each, e.g. respective) leg of a subject. The method may comprise securing one or more (e.g. each) planar section, optionally with a connector, e.g. with a tie strip. The tie strips may be detachable (e.g. removable), e.g. by tearing along a further line of weakness for each respective tie strip. It will be understood that a tie strip that is detachable (e.g. removable) by tearing along a line of weakness is more easily detachable (e.g. removable) than parts of the apparatus which are not detachable (e.g. removable) by tearing along lines of weakness (and which for example would require the use of scissors or other tools to allow detachment (e.g. removal)).

The method may comprise detaching a detachable section (e.g. a detachable section intermediate the planar sections), optionally by tearing along a line of weakness, further optionally to thereby form an aperture in the blanket for positioning between the limbs (e.g. legs) of a subject.

The apparatus may be configured to operate at one or more temperature settings, for example wherein each temperature setting involves temperature regulated gas (e.g. air) which has been regulated to fall within a predetermined range of temperatures. For example, the apparatus may be configured to operate at one or more of a 32 °C, a 38 °C, or a 43 °C temperature setting (e.g. wherein the said setting is configured to output gas of the indicated temperature ± 2 °C, or ± 1 °C). The user may select the setting, for example in dependence on the needs of the subject or in dependence on the procedure to be carried out. The method may comprise selecting the setting for example in dependence on the needs of the subject or in dependence on the procedure to be carried out.

The method may comprise moving (e.g. folding) a region of a planar section closer (e.g. a longitudinal edge of a planar section closest) to the space (e.g. the space overlapping a longitudinal axis of the second blanket portion) over a limb, e.g. towards a region of a planar section further (e.g. a longitudinal edge of a planar section furthest) from the space (e.g. the space overlapping a longitudinal axis of the second blanket portion), for example to thereby form a sleeve which wraps around the limb. In an example, the region of the planar section closer to the space may initially be beneath the subject and the method may comprise passing the region between the legs, optionally moving the planar section to cover the top of a leg, and for example moving the region to a region of the planar section further from the space (e.g. to form a sleeve which wraps around the leg). The method may comprise fixing the region of the planar section (which was originally) closer to the space to the region of the planar section further from the space to thereby form a sleeve.

According to a further aspect, there is provided a method of manufacturing a temperature regulation apparatus as described herein, wherein the method comprises bringing a first layer and a second layer into contact. The method may comprise forming one or more seals between the first layer and the second layer (e.g. at a first location) to thereby form a first blanket portion comprising an inflatable region. The method may comprise bonding the first and second layers at a second part to thereby form a second blanket portion. The method may comprise forming one or more lines of weakness through the first and second layers at the second blanket portion to thereby form at least two mutually separable planar sections. The method may comprise providing (e.g. forming) an inlet port. The method may be a method of manufacturing a temperature regulation blanket.

The method may comprise forming one or more fold lines. The method may comprise forming one or more second, third, additional, or further lines of weakness.

The method may comprise forming a locating marker on the first blanket portion, optionally to indicate the correct positioning of the head of a subject on the first blanket portion. The locating marker may be formed integrally in the first blanket portion. For example, additional internal seals may be positioned to define the locating marker. Alternatively or additionally, the locating marker may be provided in the form of a section of the first blanket portion having a different colour to the rest of the first blanket portion. Advantageously, by proving a locating marker as described herein, there is no requirement to create an opening to serve as a locating marker, and therefore waste material is reduced.

According to the invention, forming a seal may comprise applying one or more of a variety of connection techniques, including but not limited to adhesives and heat seals.

### Description of the Drawings

An example embodiment of the present invention will now be illustrated with reference to the following Figures in which:
Figure 1A is a plan elevation view of a temperature regulation apparatus according to a first example embodiment;
Figure 1B is a plan elevation illustration of the temperature regulation apparatus of Figure 1A;
Figure 2 is a cross-sectional view of a temperature regulation apparatus according to the first example embodiment;
Figure 3 is a cross-sectional view of a temperature regulation apparatus according to a second example embodiment;
Figures 4A and 4B are flowcharts including examples of steps in a method of using a temperature regulation apparatus according to an example embodiment; and
Figure 5 is a flowchart including examples of steps in a method of manufacturing a temperature regulation apparatus according to an example embodiment.

### Detailed Description of an Example Embodiment

It will be understood by those skilled in the art that any dimensions and relative orientations such as lower and higher, above and below, and directions such as vertical, horizontal, upper, lower, longitudinal, axial, radial, lateral, circumferential, etc. referred to in this description refer to, and are within expected structural tolerances and limits for, the technical field and the apparatus and methods described, and these should be interpreted with this in mind.

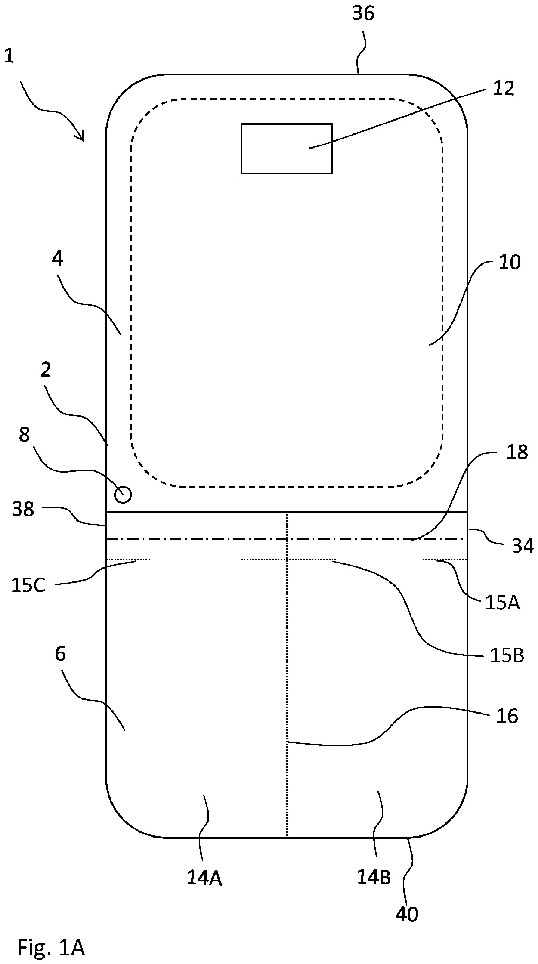

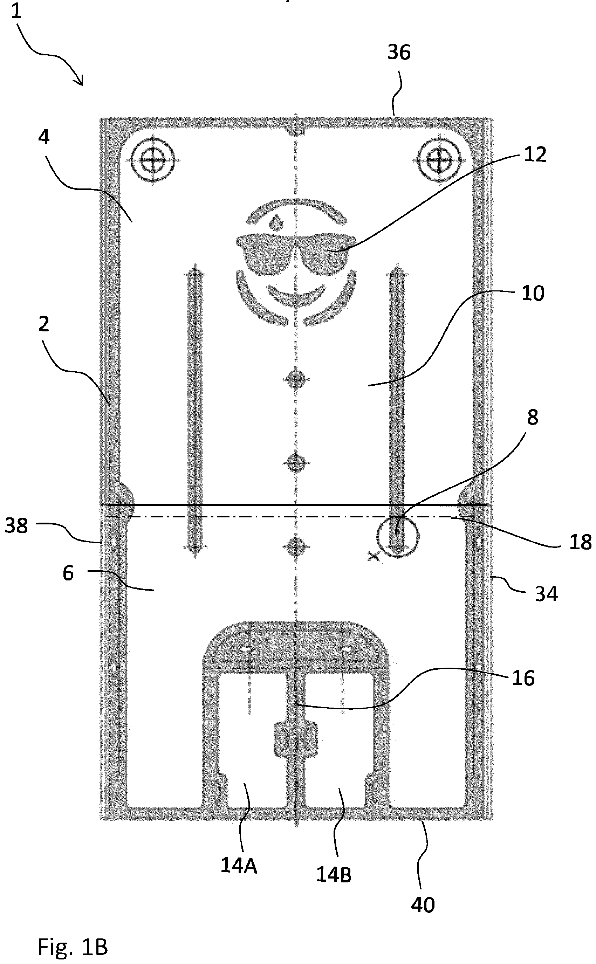

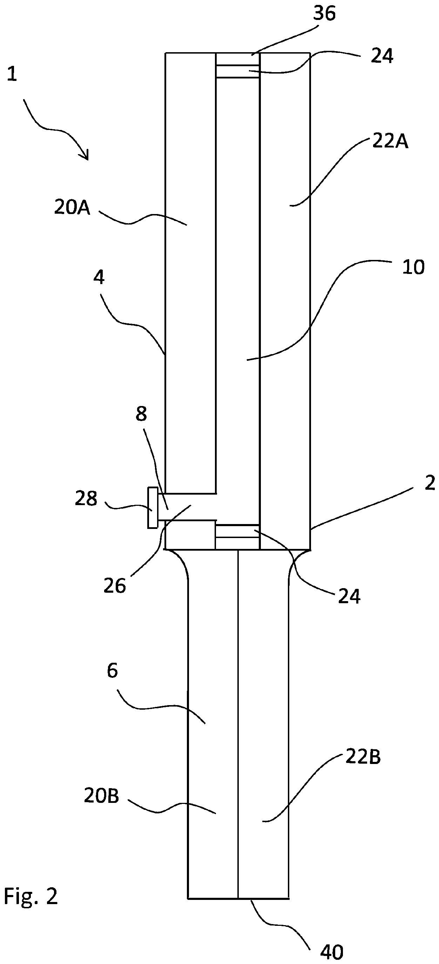

Figure 1A is a plan elevation view diagram of a first example embodiment of an apparatus 1 according to the invention, Figure 1B is an illustration of the same example embodiment, and Figure 2 is a cross-sectional view diagram of the same example embodiment of the apparatus 1. Here, the apparatus 1 is provided in the form of a temperature regulation blanket 2 in the form of an underbody. The blanket has a first, upper blanket portion 4 configured for positioning beneath a subject's upper body (e.g. including the head to at least the hips) and a second, lower blanket portion 6 configured for positioning beneath a subject's lower body (e.g. including from the hips to at least the knees). The blanket has a lowermost edge 40, a rightmost edge 34, an uppermost edge 36, and a leftmost edge 38.

The first blanket portion 4 includes an air inlet 8 in the form of an inlet port which is configured to be connectable to a supply of temperature-regulated gas (in this example, temperature-regulated air), for example via a gas supply conduit. The first blanket portion 4 also has an inflatable region 10 and a locating marker 12 (not shown in Figure 2).

The second blanket portion 6 includes first and second mutually separable planar sections 14A, 14B (not shown in Figure 2). The planar sections 14A, 14B are separable along a line of weakness 16 (not shown in Figure 2). The second blanket portion 6 also has a fold line 18 (not shown in Figure 2). In this instance, the line of weakness 16 is provided in the form of a line of perforations through the second blanket portion 6. The second planer portion 6 further includes additional lines of weakness 15A, 15B, 15C (not shown in Figure 1A), each provided in the form of a line of perforations through the second blanket portion.

As is best seen in Figure 2, the first blanket portion 4 has a first layer in the form of a first sheet 20A, the first sheet 20A being an air permeable sheet, and a second layer 22A in the form of a second sheet 22A, wherein the second sheet 22A is an air impermeable sheet, with the inflatable region 10 therebetween. It will be understood that an air impermeable layer (e.g. sheet) may still allow small quantities of air to pass therethrough at sufficient pressures, and is generally less air permeable than an air permeable layer (e.g. sheet). A seal 24 is provided between the first layer 20A and the second layer 22A around the periphery of the inflatable region 10. The first blanket portion 4 thus is provided with an air permeable region (defined by the air permeable layer over the inflatable region). This seal 24 defines the outer limits of the inflatable region 10 and prevents air from leaving other than by the first (air permeable) layer. Further seals (not shown) are formed internally within the inflatable region 10 between the first layer 20A and the second layer 22A to define a maximum separation between the first 20A and the second layer 22A and to prevent the first blanket portion 4 from ballooning excessively when temperature regulated air is supplied into the inflatable region. The air inlet 8 includes a passage 26 though the first layer 20A. The air inlet 8 also has an inlet cap 28 for closing the inlet 8 when it is not in use. The locating marker 12 is integrally formed in the first blanket portion 4.

The second blanket portion 6 also has a first air permeable layer (e.g. sheet) 20B and a second air impermeable layer (e.g. sheet) 22B, corresponding to the first and second layers 20A, 22A of the first blanket portion 4. However, the second blanket portion 6 does not have an inflatable region. Instead, in the second blanket portion 6 the first and second layers 20B, 22B are in direct contact with each other (and may for example be joined together with an adhesive layer). The perforations forming the line of weakness 16 (not shown in Figure 2) pass through both the first and second layers 20B, 22B. The second layers (e.g. sheets) 22A, 22B have a reflective outer coating (not shown). In some example embodiments, a single first, air permeable layer (e.g. sheet) 20 may extend continuously across both the first and second blanket portions, i.e. only one first air permeable layer (e.g. sheet) 20 may be provided. Similarly, in some example embodiments, a single second, air impermeable layer (e.g. sheet) 22 may extend continuously across both the first and second blanket portions, i.e. only one second air impermeable layer (e.g. sheet) 22 may be provided.

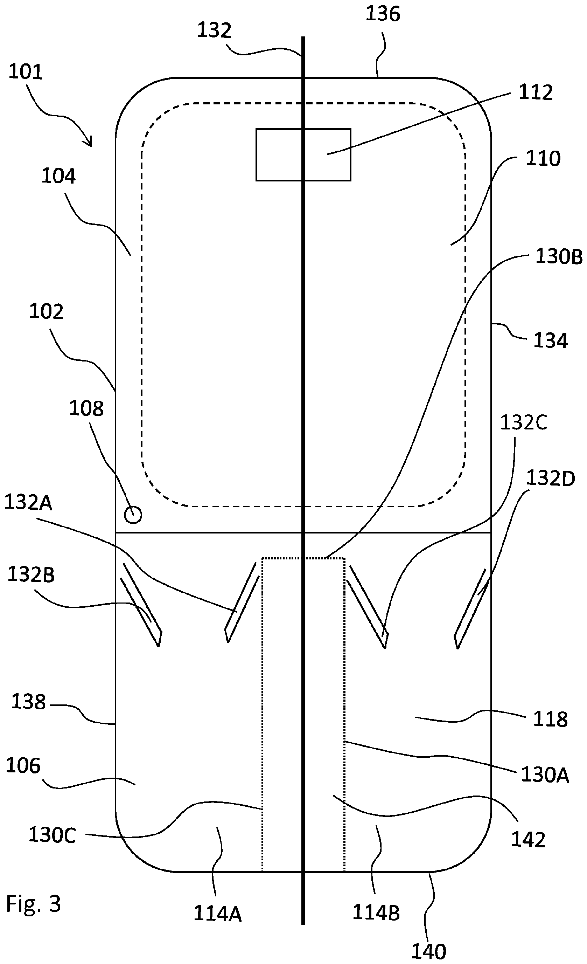

Figure 3 is a plan elevation view diagram of a second example embodiment of an apparatus 101 according to the invention. Here, the apparatus 101 is again provided in the form of a temperature regulation blanket 102 in the form of an underbody. The blanket has a first, upper blanket portion 104 configured for positioning beneath a subject's upper body (e.g. including the head to at least the hips) and a second, lower blanket portion 106 configured for positioning beneath a subject's lower body (e.g. including from the hips to at least the feet). The blanket has a lowermost edge 140, a rightmost edge 134, an uppermost edge 136, and a leftmost edge 138.

As in the case with the example embodiment of Figures 1 and 2, the first blanket portion 104 includes an air inlet 108 in the form of an inlet port (generally corresponding to and formed as that described in respect of Figures 1 and 2). The first blanket portion 104 also has an inflatable region 110 (likewise generally corresponding to and formed as that described in respect of Figures 1 and 2) and a locating marker 112. The locating marker 112 is integrally formed in the first blanket portion 104.

The second blanket portion 106 again includes first and second mutually separable planar sections 114A, 14B. The planar sections 114A, 114B are again separable along a line of weakness 116, in this case a line of weakness formed of three line portions 130A, 130B, 130C, which together form a generally u-shaped path.

The first and third line portions 130A, 130C are parallel to the central longitudinal axis 132. The first line portion 130A is off-set from the central longitudinal axis by approximately 10 centimetres, making it closer to the rightmost edge 134 of the blanket 102 than it is to the leftmost edge 138. The second line portion 130B is perpendicular to the longitudinal axis 132 and also perpendicular to the thickness dimension of the blanket, and is approximately 10 centimetres from the location where the lower blanket portion 106 meets the upper blanket portion 104. The third line portion 103C is off-set from the central longitudinal axis by approximately 10 centimetres in the opposite direction to that of the first line portion 130A, making it closer to the leftmost edge 138 of the blanket 102 than it is to the rightmost edge 134. Each line portion 130A, 130B, 130C is provided by a line of perforations through the first and second layers (e.g. sheets) (not shown in Figure 3). The line portions 130A, 130B, 130C and the lowermost edge 140 may thus be seen to define a removable section 142 intermediate the planar sections 114A, 114B.

In the example embodiment of Figure 3, the lower blanket portion 106 is also provided with connectors in the form of tie strips 132A, 132B, 132C, 132D. Each tie strip 132A, 132B, 132C, 132D is a short strip of fabric (e.g. 20 cm in length, 2 cm in width, and 2 mm in depth) and each extends from a respective upper corner region of a planar section 114A, 114B. In alternative embodiments connectors (e.g. tie strips) may additionally or alternatively be provided in the lower corner regions of a planar section 14A, 14B, 114A, 114B or indeed elsewhere on the blanket 2, 102.

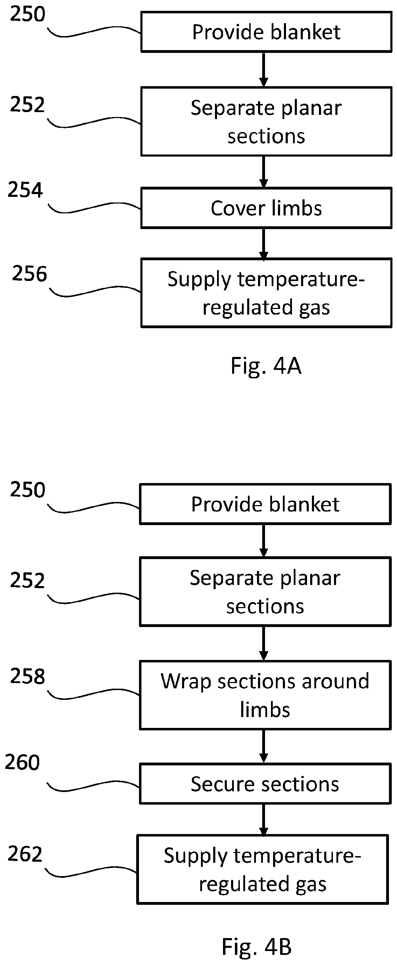

In use, and with reference to Figures 4A and 4B, an apparatus 1, 101 (e.g. a blanket 2, 202) is provided 250 in a medical setting, for example in a hospital or clinic room. In an example, the apparatus 1, 101 (e.g. the blanket 2, 202) is provided 250 on a hospital bed or lithotomy chair, such that the blanket 2, 202 may be positioned under a subject such that the first, upper blanket portion 4, 104 is beneath the subject's upper body, with the subject's head positioned over the locating marker 12, 112. The air permeable layer 20A is typically facing upwards and towards the subject.

The planar sections 14A, 14B, 114A, 114B are separated 252. In the examples of Figures 1 to 3, the planar sections 14A, 14B, 114A, 114B are separated 252 by tearing along the lines of weakness 16, 130A, 130B, 130C, through the perforations. In the case of the example of Figure 3, the removable section 142 may be removed completely.

The planar sections 14A, 14B, 114A, 114B are then used to at least partially cover the limbs 254 (in this example, the leg, in particular the upper leg, e.g. the thigh) and optionally may be wrapped at least partially around the limbs 258 (in this example, the leg, in particular the upper leg, e.g. the thigh). In some example embodiments, the sections 14A, 14B, 114A, 114B are secured 260 in place. For example, where the sections 14A, 14B, 114A, 114B are wrapped around the limbs 258 they might be secured in such as way as to prevent them from becoming unwrapped. In some instances, where the apparatus is used to cover a subject who is sitting in a lithotomy chair (or similar) the sections 14A, 14B, 114A, 114B may be secured both around the limb and to a stirrup of the lithotomy chair. Where connectors such as tie strips 132A, 13B, 132C, 132D are provided, these connectors may be used to secure 260 the sections 14A, 14B, 114A, 114B. Where provided, the additional lines of weakness 15A, 15B, 15C may also be torn along to provide greater movement for the planar sections 14A, 14B, 114A, 114B so that they can be more easily used to cover 254 or wrap around 258 the limbs of the subject.

When the sections 14A, 14B, 114A, 114B have been used to cover 254 or wrap around 258 the limbs (and optionally have been secured 260 in place), temperature-regulated gas (in this example air) is supplied 256 into the inflatable region 10, 110 via the inlet 8, 108 (typically via an air supply conduit which is connected to the inlet). This causes the inflatable region 10, 110 to inflate until the limits of the seals 24 prevent further separation of the first and second layers 20A, 22A of the upper blanket portion 4, 104 (and thus prevent further inflation). At this point, further temperature-regulated air can only enter the inflatable region 10, 110 if some air first leaves (e.g. is forced to leave) the inflatable region 10, 110. Since air is being supplied via the inlet 8, 108, it is not possible for air to leave via the inlet 8, 108. Instead, air leaves via the first, air permeable layer 20A.

As the blanket 2, 102, is positioned such that the first, upper blanket portion 4, 104 is beneath the subject's upper body, and the air permeable layer 20A is facing upwards and towards the subject, this causes temperature regulated air leaving the inflatable region (i.e. forced out of the inflatable region via the air permeable layer 20A) to be directed towards the subject and thus regulates the temperature of the subject, in particular the subject's upper body. Furthermore, temperature regulated air flows towards the uppermost edge 36, 136, the leftmost edge 38, 138, the rightmost edge 34, 134, and the second, lower blanket portion 6, 106. The air flowing towards the second, lower blanket portion 6, 106 tends to flow along the planar sections 14A, 14B, 114A, 114B and thus towards and around the subject's limbs (typically legs) thereby regulating the temperature of the subject and in particular the subject's limbs (and thus lower body). Meanwhile, the reflective outer coating passively reflects heat back towards the subject, thus offering further temperature regulation.

When temperature regulation is no longer required, the temperature regulated air supply can be disconnected from the inlet 8, 108 and the inflatable region 10, 110 can be allowed to naturally deflate. The sections 14A, 14B, 114A, 114B can be removed from and/or unwrapped from around the subject's limbs.

Should the planar sections 14A, 14B, 114A, 114B be in the way they can be folded out of they way along fold line 18. Should the planar sections 14A, 14B, 114A, 114B not be needed, it is possible to not separate the sections 14A, 14B, 114A, 114B and to then use the blanket 2, 102 in the normal way, either as an overbody blanket (over the subject) or as an underbody (under the subject). This is helpful, as it reduces the likelihood of the sections 14A, 14B, 114A, 114B getting in the way, or getting folded uncomfortably under the subject.

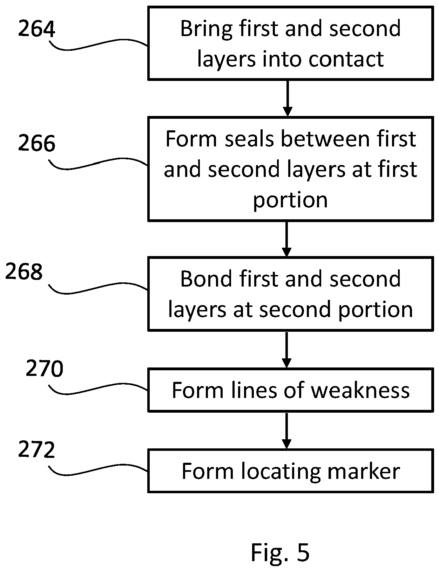

With reference to Figure 5, the apparatus 1, 101 (e.g. the blanket 2, 102) is formed by bringing 264 the first, air permeable layers 20, 20A, 20B into contact with the second layers 22, 22A, 22B. Seals are then formed 266 between the first 20, 20A and second 22, 22A layers at the first blanket portion 4, 104 to thereby form the inflatable region 10, 110. This includes the internal seals which prevent the inflatable region 10, 110 from ballooning excessively in use. The first 20, 20B, and second 22, 22B layers at the second blanket portion 6, 106 are bonded 268 together, without an inflatable region being formed in the second blanket portion 6, 106. Lines of weakness 16, 116 (e.g. perforations) are then formed in the second blanket portion 6, 106, to thereby define the planar sections 14A, 14B, 114A, 114B and allow their subsequent mutual separation. Where the lines of weakness 16, 116 are provided by perforations, these perforations are formed through both the first and second layers 20, 20B, 22, 22B. A locating marker 12, 112 is formed 272 integrally in the first blanket portion 4, 104, for example by the addition of further seals, or by the application of a pigmented section (e.g. through the use of a dye) at the region where the subject's head should be positioned. Although not shown in Figure 5A, an air inlet is formed in the blanket 2, 102, by piercing a hole in the first layer 20, 20A and into the inflatable region 10, 110, and providing a valve and cap at the first layer to thereby allow connection to a gas supply conduit.

In this example embodiment, the first, air permeable layer 20, 20A, 20B is fabricated of polypropylene. The second, air impermeable layer 22, 22A, 22B is fabricated of polyethylene, with a reflective outer layer. The seals are formed by heat sealing the two layers. At the second blanket portion 6, 106, the two layers are joined together with a layer of adhesive.

In the example embodiments of Figures 1 to 3, the blanket has a length of 2 meters from the uppermost edge 36, 136 to the lowermost edge 40, 146 and a width of 1.25 meters from the leftmost edge 38, 138 to the rightmost edge 134 (e.g. when the inflatable region 10, 110 is uninflated). The second blanket portion 6, 106 has a length of 0.8 meters, extending from the upper blanket portion 6, 106 to the lowermost edge 40, 140. Accordingly, the second blanket portion 6, 106 provides planar sections which are sized and shaped such that they can (at least partially) cover or be wrapped around a subject's upper leg, where the subject has legs of a dimension typical for an adult human. When inflated, the first and second layers 20, 22 at the inflatable region 10, 110, are separated by a maximum distance of 10 centimetres.

Although the apparatus 1, 101 in the examples shown in Figure 1 to 3 is envisioned as an underbody (i.e. the blanket 2, 102 would be positioned beneath a subject) this is not required, and the apparatus may in the alternative be positioned over a subject. Furthermore, while a fold line 18 may be provided to allow for the planar sections 14A, 14B to be moved out of the way, in some examples, a further line of weakness may be provided instead of or in addition to the fold line 18, to allow the planar sections to be detached from the blanket 2, 102.

Although the example embodiment of the apparatus 1, 101 provides a second blanket portion 6, 106 with planar sections 14A, 14B, 114A, 114B that are sized and shaped to at least partially cover (or be at least partially wrapped around) the upper part of a subject's leg, this is not required. They may in the alternative be sized and shaped to at least partially cover (or be at least partially wrapped around) a part or whole of either an arm or a leg. Furthermore, larger or smaller blankets 2, 102 and planar sections 14A, 14B, 114A, 114B are envisioned for use for larger and smaller subjects, and the skilled person will appreciate that variations in dimension and shape are within the scope of the invention.

In summary, there is provided a temperature regulation apparatus (1, 101) comprising a forced gas temperature regulation blanket (2, 102) for providing temperature-regulated gas to a subject, the forced gas temperature regulation blanket comprising: a first blanket portion (4, 104) comprising: an inflatable region (10, 110), the inflatable region comprising a gas permeable region; and an inlet port (8, 108) for receiving temperature-regulated gas into the inflatable region, a second blanket portion (6, 106) comprising at least two at least partially mutually separable planar sections (14A, 14B, 114A, 114B), each configured for covering a limb of a subject.

Throughout the description and claims of this specification, the words "comprise" and "contain" and variations of them mean "including but not limited to", and they are not intended to and do not exclude other components, integers, or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment, or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

## Claims

1. A temperature regulation apparatus comprising a forced gas temperature regulation blanket for providing temperature-regulated gas to a subject, the forced gas temperature regulation blanket comprising:
a first blanket portion comprising:
an inflatable region, the inflatable region comprising a gas permeable region; and
an inlet port for receiving temperature-regulated gas into the inflatable region,
a second blanket portion comprising at least two at least partially mutually separable planar sections, each configured for covering a limb of a subject.

2. A temperature regulation apparatus according to claim 1, wherein the second blanket portion is configured such that separation of the mutually separable planar sections defines a space in the second blanket portion, the space overlapping a longitudinal axis of the second blanket portion.

3. A temperature regulation apparatus according to claim 1 or claim 2, wherein the first blanket portion comprises an underbody.

4. A temperature regulation apparatus according to any one preceding claim, wherein the second blanket portion comprises at least one first line of weakness, configured to form the at least two mutually separable planar sections.

5. A temperature regulation apparatus according to any one preceding claim, wherein the second blanket portion comprises a fold line configured to allow the planar sections to be folded towards the torso of the subject, optionally wherein the fold line comprises at least one second line of weakness, configured to allow the planar sections to be detached from the blanket.

6. A temperature regulation apparatus according to any one preceding claim, comprising:
a first layer; and
a second layer,
wherein the first layer is a gas permeable layer and the forced gas temperature regulation blanket is configured to allow gas to leave the inflatable region via the first layer and thereby be directed towards a subject.

7. A temperature regulation apparatus according to claim 6, wherein the first blanket portion comprises one or more seals formed between the first layer and the second layer, the one or more seals at least partially bounding the inflatable region.

8. A temperature regulation apparatus according to any one preceding claim, wherein the second blanket portion comprises one or more connectors, each of the one or more connectors configured to secure a respective planar section at least partially cover the limb of a subject.

9. A temperature regulation apparatus according to any one preceding claim, wherein the forced gas temperature regulation blanket either:
comprises a detachable section boarded by at least one third line of weakness such that the detachable section can be detached from the blanket to thereby form an aperture in the blanket for positioning between the limbs of a subject; or
has an aperture defined therein, the aperture for positioning between the limbs of a subject.

10. A temperature regulation apparatus according to any one preceding claim, wherein the first blanket portion comprises a locating marker configured to indicate where the blanket should be positioned relative to a subject.

11. A method of supplying temperature-regulated gas to a subject using a temperature regulation apparatus according to any preceding claim, the method comprising;
providing the forced gas temperature regulation blanket beneath a subject; and
causing temperature-regulated gas to be supplied into the inflatable region.

12. A method according to claim 11, further comprising:
separating the mutually separable planar sections; and
at least partially covering the limb of a subject with a planar section.

13. A method according to claim 11 or claim 12, wherein the second blanket portion comprises one or more connectors, the one or more connectors configured to secure a planar section around the limb of a subject,
and wherein the method comprises securing a planar section at least partially around a limb of the subject,
optionally wherein the method comprises securing the planar section at least partially around both the said limb and at least one further structure.

14. A method of manufacturing a temperature regulation apparatus according to any one of claims 1 to 10, the method comprising:
bringing a first sheet and a second sheet into contact;
forming one or more seals between the first sheet and the second sheet at a first part to thereby form a first blanket portion comprising an inflatable region;
bonding the first and second sheets at a second part to thereby form a second blanket portion; and
forming one or more lines of weakness through the first and second sheets at the second blanket portion to thereby form at least two mutually separable planar sections.

15. A method according to claim 14, comprising forming a locating marker on the first blanket portion to indicate the correct positioning of the head of a subject on the first blanket portion.
